# EUROPEAN PATENT SPECIFICATION

(11) **EP 1 069 099 B1**
(45) Date of publication and mention of the grant of the patent: **16.03.2005**
(21) Application number: 00202433.9
(22) Date of filing: 10.07.2000
(51) Int. Cl.: C07C 2/86, C07C 39/04

(54) **Process for the alkylation of aromatic compounds in gas phase**
Verfahren zur Alkylierung von aromatichen Verbindungen in der Gasphase
Procédé pour l'alkylation de composés aromatiques en phase gazeuse

(30) Priority: 13.07.1999 IT MI991530
(43) Date of publication of application: 17.01.2001
(73) Proprietor: Polimeri Europa S.p.A., 72100 Brindisi (IT); ENITECNOLOGIE S.p.A., 20097 S. Donato Milanese (Milano) (IT)
(72) Inventor: Girotti, Gianni, 28100 Novara (IT); Pollastri, Massimiliano, 40139 Bologna (IT); Rivetti, Franco, 20139 Milan (IT); Perego, Carlo, 20040 Carnate (Milan) (IT)
(74) Representative: De Gregori, Antonella

(56) References cited:
- EP-A- 0 538 518
- US-A- 5 015 786

## Description

The present invention relates to a process for the alkylation of benzene with the alkylating agent isopropanol (IPA), or mixtures of isopropanol and propylene, under pressure and temperature conditions corresponding to complete gas phase of the mixture present in the reaction section and in the presence of a catalyst comprising beta zeolite and an inorganic binder characterized by an extra-zeolitic porosity consisting, for a fraction of at least 25%, of pores with a radius higher than 100 Angstrom, wherein when the alkylating agent is a mixture of isopropanol and propylene the molar ratio between benzene and alkylating agent ranges from 3 to 10 and the molar ratio between isopropanol and propylene ranges from 10 to 0.01.

In fact an aspect of the present invention is to use catalytic compositions comprising the beta zeolites described in EP 687,500 and EP 847,802, i.e. catalytic compositions consisting of beta zeolite and an inorganic ligand characterized by an extra-zeolite porosity consisting, for a fraction of at least 25%, of pores with a radius exceeding 100 Å and, in the case of EP 847,802, characterized by a total extra-zeolitic porosity volume greater than or equal to 0.80 ml/g.

The process is characterized by the total absence of negative effects on performance and duration of the catalyst due to the presence of high quantities of water in the reaction mixture.

The absence of these negative effects is due to the catalytic system used which proves to be particularly suitable for the alkylation of benzene with the alkylating agent isopropanol or mixtures or isopropanol and propylene.

The invention also relates to the process for preparing phenol in which the first preparation step of cumene is effected by the alkylation of benzene according to what is specified above.

Cumene is an important precursor for the production of phenol, which in turn is useful as an intermediate in the preparation of caprolactam from which nylon is produced.

The complete preparation process of phenol comprises the alkylation of benzene to cumene, the oxidation of cumene to the corresponding hydroperoxide which, by acid treatment, generates phenol and acetone.

With respect to the first alkylation step, catalysts based on phosphoric acid and infusorial earth for fixed bed reactors or AlCl₃ in slurry, are still widely used in the petrochemical industry.

These processes however create problems relating to environmental impact and safety: in fact, the use of these catalysts is particularly problematical due to corrosion, the by-production of toxic organic products and disposal of the exhausted catalysts.

In 1965 the preparation of cumene using X zeolite and Y zeolite was described for the first time (Minachev, Kr. M., et al, Neftekhimiya 5 (1965) 676). The use of zeolites with a faujasitic structure for the alkylation of benzene with light olefins, such as propylene, was subsequently described by Venuto et al. (J. Catal.5, (1966)81).

Excellent results, in terms of industrial application, have been obtained in the synthesis of cumene using zeolites with a beta type structure, as described in EP 432,814, and in particular using catalysts comprising beta zeolite as described in EP 687,500.

EP 538518 discloses a process for the preparation of cumene which comprises reacting benzene with a propylating agent in the presence of a catalyst containing metal loaded beta zeolite wherein the propylating agent is selected from propylene and propyl alcohol. Beta zeolite can be incorporated in matrix materials such as clay, silica, alumina or metal oxides. EP538518 does not teach any characteristics of porosity of the used catalytic composition.

Once obtained, cumene is transformed into phenol by means of an oxidation step to cumylhydroperoxide, followed by an acid treatment step which causes breakage of the peroxide bond with the formation of phenol and acetone.

If, on the one hand, the simultaneous production of phenol and acetone in a single productive unit is certainly a positive aspect from an industrial point of view, on the other hand the existence of an unbalanced commercial demand for the two products can cause problems in running an industrial plant for the production of phenol.

It should, in fact, be remembered that for every Kg of phenol produced from Cumene according to the traditional process via propylene, 0.61 Kg of acetone are also obtained. Considering that one of the main uses of acetone is represented by methylmethacrylate (MMA) whose market demand is decreasing, whereas the demand for bisphenol A (BPA), phenolic resins and caprolactam, the main uses of phenol, is growing, the potential problem deriving from the coproduction of acetone in the production process of Phenol via Cumene, is understandable. There is consequently a great necessity for finding a possible alternative use which enables acetone to be conveniently exploited when market conditions are not favourable towards direct sales.

U.S. 5,017,729 describes a process for the production of phenol via cumene hydroperoxide characterized by the use of propylene, in the preparation step of cumene, either totally or partially deriving from the reduction with hydrogen of acetone (co-produced with phenol) and subsequent dehydration of isopropylic alcohol.

The high costs of the various steps for re-obtaining pure propylene - to be used in the alkylation step - starting from the acetone co-produced with phenol, are evident in this process.

In particular, in the process proposed by Mitsui (PEP Review 95-1-11) for the production of propylene starting from acetone, it appears that the higher investment costs can be attributed to the dehydration section of isopropylic alcohol - obtained from acetone in the relative reduction section with hydrogen - to propylene.

It is known, in fact, that the dehydration step of IPA to propylene is necessary, for concrete industrial application, due to the impossibility of carrying out the alkylation of benzene directly with isopropylic alcohol as alkylating agent, when acid catalysts of the conventional type are used, as a result of the water released from IPA during the reaction, which has negative effects on the catalyst performance in terms of selectivity and, above all, duration of the catalyst itself.

Acid catalysts, both of the zeolitic and non-zeolitic type, are in fact negatively influenced by the presence of water which is developed when isopropylic alcohol is used as alkylating agent of benzene to give cumene. In the case of a catalyst of the conventional type such as, for example, phosphoric acid supported on silica, widely used in the industrial synthesis of cumene, quantities of water higher than a few hundred ppm in the reaction mixture produce a significant chemical and mechanical disgregation of the catalyst together with a considerable lowering of catalytic performances in terms of yield to cumene.

In the case of catalysts based on zeolites, the negative effect due to the presence of water which causes a lowering of the overall yield to cumene, together with a more or less rapid deactivation of the catalyst itself, is known. All these negative effects are known and verified also with very low contents of water - present in the reaction - with respect to those caused by the use of isopropylic alcohol as alkylating agent of benzene to give cumene in a process applicable on an industrial scale.

The industrial applicability of an alkylation process of benzene with isopropylic alcohol cannot, in fact, omit to take into account certain parameters such as for example the molar ratio benzene/IPA in the feeding to the reaction section, which generally ranges from 4 to 8 with a corresponding concentration of water in the reaction equal to about 48,000 and 26,000 ppm, assuming the total conversion of isopropylic alcohol.

Even if the alkylation of benzene were carried out with an alkylating agent consisting of a mixture of isopropanol and propylene, in any case a considerable reduction in the quantity of isopropylic alcohol used would be required to guarantee a water content which could be tolerated by the catalytic system, thus limiting the potentiality of the process itself.

The catalysts used for the alkylation of benzene with propylene are not always easily adaptable to the alkylation reaction of benzene with isopropylic alcohol, or mixtures of isopropylic alcohol and propylene, as alkylating agent, as these catalysts are generally very sensitive to water and consequently their life in the presence of water formed by the dehydration of isopropanol, is very reduced.

The possibility of alkylating benzene with isopropylic alcohol using a beta zeolite as catalyst, in gas phase, preferably at atmospheric pressure has also been described (K.S.N.Reddy et al., Applied Catalysis A: General, 95 (1993) 53-63). Also in this case the deterioration of the catalyst, also observed with high benzene/isopropanol ratios, is evident.

In the experimental tests described in the above reference, problems relating to the duration of the catalyst arise as the experimental tests proceed.

U.S. 5,015,786 describes a production process of phenol via cumene in which part of the cumene derives from the alkylation of benzene, also effected with isopropylic alcohol obtained by the reduction of the acetone co-produced with phenol, together with cumene deriving from the alkylation of benzene with propylene.

The alkylation step of benzene with IPA is carried out in the presence of a catalyst of an acidic nature, selected from various materials: zeolites are indicated as preferred catalysts. It is interesting to note however that, in the above document, there is no information regarding the life of the catalyst and constancy of the performances in general due to the fact that the test with the longest duration lasts 200 hours (Example 5, column 15) which correspond, under the conditions specified, to a productivity not higher than about 100 Kg of Cumene/Kg of catalyst.

The avoid the problems mentioned above, the use of particular zeolites with distinct hydrophobic characteristics has been proposed, such as ZSM-5 zeolite with a high silica/alumina ratio or dealuminated H-mordenite and Y zeolite.

For example, in U.S. 5,160,497 a dealuminated Y zeolite is used, with a molar ratio SiO₂/Al₂O₃ ranging from 8 to 70, for the alkylation of benzene with propylene and isopropanol.

We have now found that it is possible to obtain cumene by the alkylation of benzene with IPA, or mixtures of IPA and propylene as alkylating agent, by means of a process which provides better performances and above all catalyst duration, even in the presence of large quantities of water, operating under pressure and temperature conditions corresponding to complete gas phase of the mixture present in the reaction section and in the presence of a catalyst comprising beta zeolite and an inorganic binder characterized by an extra-zeolitic porosity consisting, for a fraction of at least 25%, of pores with a radius higher than 100 Angstrom, wherein when the alkylating agent is a mixture of isopropanol and propylene the molar ratio between benzene and alkylating agent ranges from 3 to 10 and the molar ratio between isopropanol and propylene ranges from 10 to 0.01.

The process according to the present invention allows any molar ratio between benzene and isopropylic alcohol to be used in the feeding to the reaction section, in a range of concrete industrial applicability, and consequently regardless of the total quantity of water developed during the reaction.

A particularly preferred aspect of the present invention is to use catalytic compositions comprising beta zeolite already described in EP 687,500 and EP 847,802, i.e. catalytic compositions consisting of beta zeolite and an inorganic ligand characterized by an extra-zeolite porosity consisting, for a fraction of at least 25%, of pores with a radius higher than 100 Å and, in the case of EP 847,802, also characterized by a total extra-zeolitic porosity volume greater than or equal to 0.80 ml/g.

According to a preferred aspect of the present invention, the process is carried out at a reaction temperature ranging from 150°C to 230°C, at a reaction pressure ranging from 1 to 20 bars and under such conditions, however, as to have the reaction mixture present in completely gas phase and indifferently using isopropanol or mixtures of isopropanol and propylene as alkylating agent.

In the process claimed herein, the molar ratio between benzene and isopropanol preferably varies from 3 to 10, even more preferably from 4 to 8.

When propylene is also additionally used as alkylating agent together with isopropanol, the molar ratio between benzene and alkylating agent isopropanol plus propylene varies from 3 to 10, more preferably from 4 to 8, and the molar ratio between isopropanol and propylene varies from 10 to 0.01 and even more preferably from 5 to 0.1.

The alkylation of benzene with isopropanol can be carried out in continuous, semi-continuous or batchwise.

When the process is carried out in continuous, it is also possible to use a configuration of the reaction system which comprises the partial recycling of the effluent of the reaction section, after cooling and separation of the aqueous phase from the organic phase, to the reaction section itself.

The alkylation reaction of benzene with IPA or mixtures of IPA and propylene as alkylating agent remains however exothermal, in spite of the presence of IPA, and in order to maintain the temperature within a preferred range and reduce the by-production of aromatic polyalkylated products, the catalyst can be distributed in the reactor in various layers inside a fixed bed reactor.

A quench is effected between one layer and another with inert solvents and/or part of the benzene and/or part of the isopropylic alcohol or mixture of isopropylic alcohol/propylene as alkylating agent.

Operating conveniently, high benzene/alkylating agent ratios can be obtained on the single layer, without increasing the same overall ratio, which is an obvious advantage with respect to the selectivity to cumene and consequently the separation operations downstream of the reaction section.

The temperature control can be effected not only by means of quenching the reagents and/or inert products, but also by means of inter-cooling between the layers.

The alkylation reaction can be suitably carried out in two or more reactors in series, inter-cooled to control the temperature. The feeding of the isopropylic alcohol, optionally mixed with propylene and/or benzene, can be suitably divided between the various reactors and reactor layers, i.e. the alkylating agent and/or benzene are added in more than one step.

Also included in the scope of the present invention is a process for the preparation of phenol comprising the following steps:
1) alkylation of benzene with isopropylic alcohol, and optionally propylene, to give cumene and water;
2) oxidation of the cumene thus obtained;
3) treatment of cumylhydroperoxide with acids to obtain a mixture of phenol and acetone;
4) hydrogenation of the acetone to isopropanol which is recycled to step 1.
In particular:
1) the alkylation of benzene with isopropylic alcohol, and optionally propylene, to give cumene and water, is carried out in the presence of a catalyst based on beta zeolite and, preferably, with a catalyst prepared according to the procedure described in EP 687,500 and EP 847,802 under pressure and temperature conditions which are such that the reaction mixture is present in completely gaseous phase;
2) the cumene deriving from step 1 is oxidized with air to give cumyl hydroperoxide, which in turn is treated with an acid to give a mixture of phenol and acetone which is fractionated to separate the phenol from the acetone;
3) the acetone obtained in step 2 is partly or totally hydrogenated to isopropylic alcohol which is recycled to step 1.

According to a preferred aspect, at the end of the first step, after separating by fractionation the desired product, cumene, which passes to the subsequent oxidation step, the remaining fraction of polyisopropylbenzenes is used in a separate step for a transalkylation reaction with benzene to recover additional cumene.

The transalkylation reaction is carried out in the presence of beta zeolite or a catalyst based on beta zeolite, in particular prepared according to the procedure described in EP 687,500 and EP 847,802.

The temperature conditions for the transalkylation reaction are selected from 100°C to 350°C, the pressure is selected from 1.013 MPa to 5.065 MPa (10 to 50 atm) and the WHSV ranges from 0.1 h⁻¹ to 200 h⁻¹. These conditions are already described in EP 687,500.

Consequently, in step (2), the cumene deriving from step (1), and optionally from the transalkylation step, is oxidized to cumyl hydroperoxide. The cumyl hydroperoxide is then transformed into phenol and acetone. In the last step, part of or all the acetone obtained as by-product in step (2) is hydrogenated to isopropylic alcohol which is re-fed to the initial step.

The hydrogenation reaction of acetone to isopropanol is already known and is carried out using catalysts based on Nickel Raney, nickel-copper, copper-chromium, copper-zinc or based on metals of the platinum group for example platinum, palladium, ruthenium, rhodium.

A catalyst based on Nickel Raney or copper-chromium is preferably used.

The conditions under which the hydrogenation reaction of acetone takes place are described, among others, in U.S. 5,015,786 or U.S. 5,017,729.

A remarkable aspect of the processes claimed herein and in particular of the alkylation step of benzene with isopropanol or mixtures of propylene and isopropanol, is the great flexibility in re-using the acetone co-produced with phenol, from which isopropylic alcohol is obtained by reduction with hydrogen. This flexibility is in fact obtained by the use, in our process, of the catalyst based on beta zeolite prepared according to the procedure described in EP 687,500 and EP 847,802, which guarantees the absence of performance reduction and rapid deactivation phenomena typical of solid acid catalysts due to the presence of water generated by the use of isopropylic alcohol as alkylating agent of benzene.

The purpose of the following examples is to illustrate the invention claimed herein, without limiting however its scope in any way.

### EXAMPLE 1

An alkylation test of benzene is carried out with isopropylic alcohol using the experimental device described hereunder.

The experimental device consists of tanks for the benzene and isopropylic alcohol reagents, feeding pumps of the reagents to the reactor, pre-heating unit of the reagents, steel reactor situated inside an electric heating oven, regulation loop of the internal temperature of the reactor, regulation loop of the internal pressure of the reactor, reactor effluent cooler and collection system of the liquid and gaseous products.

In particular, the reactor consists of a steel cylindrical tube with a mechanical sealing system and diameter of about 2 cm.

A thermometric holder having a diameter of 1 mm is situated along the major axis of the reactor, inside which there is a thermocouple free to run along the major axis of the reactor.

A catalyst based on beta zeolite, prepared according to the procedure described in patent application EP 847,802, is charged into the reactor in a quantity corresponding to a height of the catalytic bed equal to 10 cm.

A quantity of inert material is charged on top of and below the catalytic bed to complete the bed.

The benzene and isopropanol (IPA) reagents - preheated and premixed in a suitable mixer - are fed to the reactor with up flow.

The reaction products, liquid and gaseous, are analyzed through gaschromatography using the following equipment:
(a) Carlo Erba GC 6000 gaschromatograph equipped with a MEGA SE54 column having an external diameter of 0.53 mm and a length of 25 m, FID detector and temperature program;
(b) HP 6890 gaschromatograph equipped with a PONA column having an external diameter of 0.2 mm and a length of 50 m, FID detector and temperature program;
(c) Carlo Erba 4200 gaschromatograph equipped with a Poro-pack-Q filled column having a diameter of 4 mm and a length of 2 m, TCD detector and temperature program.

The reaction conditions at which the test was carried out are as follows:

| | |
|---|---|
| Reaction temperature | 190°C |
| Reaction pressure | 1 bar |
| WHSV | 4 h⁻¹ |
| [Benzene]/[IPA] in feeding | 5.82 moles/moles |

These conditions ensure that the reaction system is in gas phase.

The attribution of the physical state of the reagent mixture is effected both by comparison with the existing phase diagrams for the components and mixtures in question, and also by calculation, adopting the RKS state equation (Soave G. Chem. Eng. Sci 27, 1197, (1972)). The interaction parameters for this equation are obtained from regression of the experimental data in literature relating to liquid-vapor equilibrium and mutual solubilities of the hydrocarbon-water mixtures (C.C. Li, J.J. McKetta Jul. Chem. Eng. Data 8 271-275 (1963) and C. Tsonopoulos, G.M. Wilson ALCHE Journel 29, 990-999, (1983).

The reaction system to which the above equation is applied resembles, as far as the compositions are concerned, the system [benzene]/[propylene] = 5.8 and [benzene]/[water] = 5.8.

The total water concentration present in the system, with complete conversion of the isopropylic alcohol reagent, is equal to 35,000 ppm.

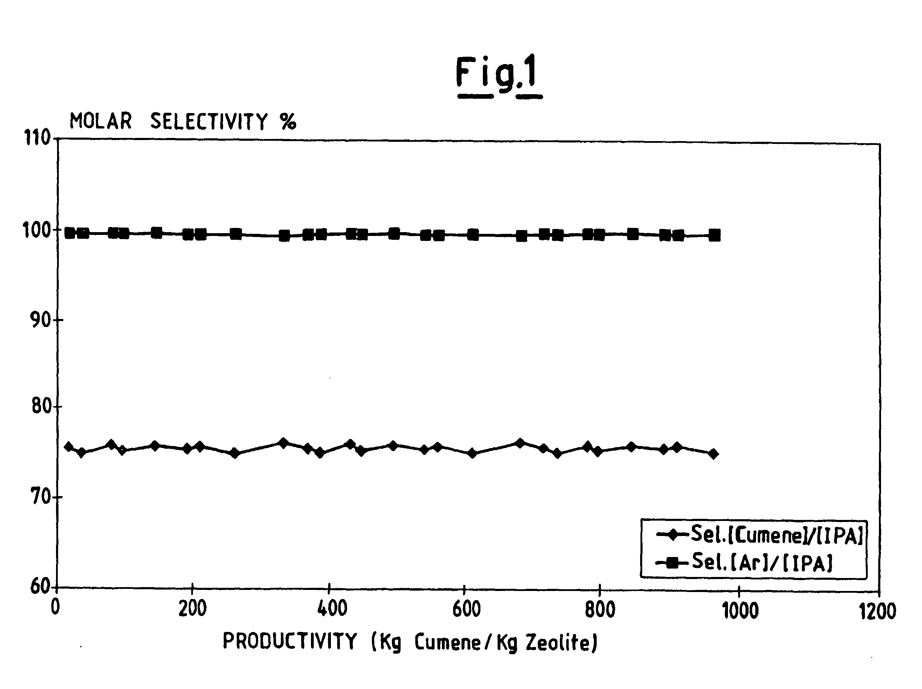

Figure 1 shows the trend of the molar selectivity [Ar]/[IPA] (Cumene + Diisopropylbenzenes + Triisopropylbenzenes with respect to the total IPA converted) in relation to the productivity of the catalyst expressed in Kg cumene/Kg beta zeolite and the trend of the molar selectivity [Cum]/[IPA] (Cumene with respect to the total IPA converted) in relation to the productivity of the catalyst in Kg cumene/Kg beta zeolite.

For the whole duration of the test (about 1500 hours) there were no signs of deactivation of the catalyst such as, for example, a drop in the conversion of the alcohol (not indicated in figure 1 but quantitative for the whole duration of the test) or an increase in the fraction of polyalkylated products.

The selectivities during the whole test, in fact, remained unaltered with values equal to about 75.5% for the selectivity [Cum]/[IPA] and about 99.6% for the selectivity [Ar]/[IPA].

It should be pointed out that the constancy of the two selectivity data is extremely significant as it indicates a constant catalytic activity, also with respect to the transalkylation reaction of polyalkylated products, which, as is known to experts in the field, always takes place contemporaneously with the alkylation reaction.

The deactivation of the catalyst would, in fact, have been observed, even before a drop in the conversion of the reagents, by a decrease in the selectivity [Cum]/[IPA] (also with the same [Ar]/[IPA] selectivity) due to a reduction in the catalytic activity in the transalkylation reaction of polyalkylated products.

### EXAMPLE 2

The same experimental equipment described in example 1 is used, under the same experimental conditions, except for the reaction temperature which is raised to 210°C. The catalyst used is the same as example 1.

The reaction conditions at which the test was carried out are therefore the following:

| | |
|---|---|
| Reaction temperature | 210°C |
| Reaction pressure | 1 bar |
| WHSV | 4 h⁻¹ |
| [Benzene]/[IPA] in feeding | 5.82 moles/moles |

These conditions ensure that the reaction system is in gas phase.

The attribution of the physical state of the reagent mixture is effected as described in example 1 obviously taking the different temperature into account.

The analysis of the reaction products is also carried out as described in example 1.

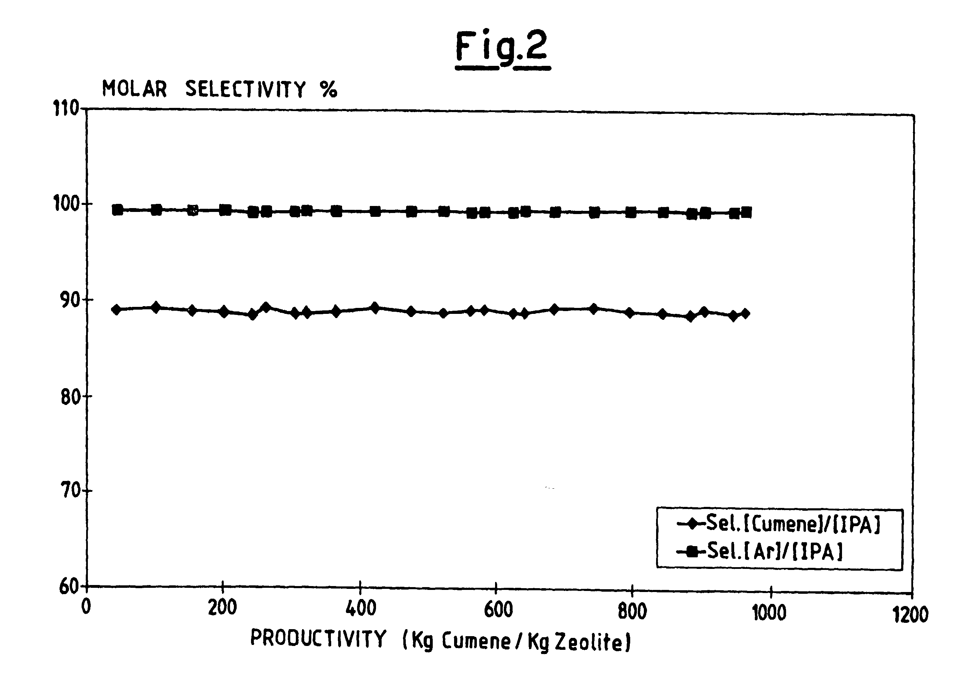

Figure 2 shows the trend of the molar selectivity [Ar]/[IPA] and of the molar selectivity [Cum]/[IPA] in relation to the productivity of the catalyst.

For the whole duration of the test (about 1250 hours) there were no signs of deactivation of the catalyst such as, for example, a drop in the conversion of the alcohol (quantitative for the whole duration of the test) or an increase in the fraction of polyalkylated products.

The selectivities during the whole test remained unaltered with values equal to about 89.0% for the selectivity [Cum]/[IPA] and about 99.4% for the selectivity [Ar]/[IPA].

The higher temperature with respect to that adopted in the previous example results in a greater contribution of the transalkylation reaction of the polyalkylated products to the [Cum]/[IPA] selectivity whose value in fact is higher than that obtained in the previous example.

The results obtained in this example can be directly compared to those indicated in Applied Catalysis A., 95 (1993) 53-63 K.S.N. Reddy, B.S. Rao and V.P. Shiralkar, on page 60, where the following reaction conditions are used:

| | |
|---|---|
| Reaction temperature | 210°C |
| Reaction pressure | 1 bar |
| WHSV | 4 h⁻¹ |
| [Benzene]/[IPA] in feeding | 3 moles/moles |

These conditions ensure that the reaction system is in gas phase.

The attribution of the physical state of the reagent mixture is effected as described above by introducing the data specified in the reference already mentioned which correspond, as far as the compositions are concerned, to the system [benzene]/[propylene] = 8 moles/moles and [benzene)/[water] = 8 moles/moles.

The above reference reaction conditions, corresponding to a reaction in gas phase, are therefore comparable to those of our test and are less drastic with respect to the molar ratio [benzene]/[IPA] in the feeding which proves in fact to be equal to 8 against a ratio equal to 5.8 in our test.

In the above reference test there is a constant drop in the [Cum]/[IPA] selectivity in relation to the time on stream and in general a constant deactivation of the catalyst as the test proceeds (Applied Catalysis A., 95 (1993) 53-63 K.S.N. Reddy, B.S. Rao and V.P. Shiralkar, on page 60, page 61).

The [Cum]/[IPA] selectivity in fact turns from about 94% to about 92%, after less than 500 hours of reaction corresponding to a productivity equal to about 350 Kg cumene/Kg beta, obtained with simple calculations from the data specified in the reference.

From the data of figure 2, it can be seen that with analogous productivity values (about 350 Kg cumene/Kg beta) there is no drop in the selectivity in the test carried out according to our invention, i.e. using the catalyst prepared according to the procedure described in EP 847,802.

The catalyst used according to our invention does not show any signs of deactivation unlike the catalyst used in the above reference, even operating with lower molar ratios in the feeding between benzene and isopropanol, which certainly represents a more drastic condition as far as the deactivation of the catalyst is concerned.

It is therefore evident that under the conditions of our invention better results are obtained in terms of reduction in the deactivation rate of the catalyst.

In the test effected in the reference cited above, there is a higher selectivity to cumene than that obtained in our experiment due, as is known to experts in the field, to the higher molar ratio between benzene and isopropylic alcohol in the feeding, which favours, as is known, a greater selectivity towards monoalkylation regardless of the total selectivity to cumene + diisopropylbenzenes + triisopropylbenzenes (Sel.[Ar.]/[IPA].

## Claims

1. A process for the alkylation of benzene with isopropanol, or a mixture of isopropanol and propylene, under pressure and temperature conditions corresponding to complete gas phase of the mixture present in the reaction section and in the presence of a catalyst comprising beta zeolite and an inorganic binder **characterized by** an extra-zeolitic porosity consisting, for a fraction of at least 25%, of pores with a radius higher than 100 Angstrom, wherein when the alkylating agent is a mixture of isopropanol and propylene the molar ratio between benzene and alkylating agent ranges from 3 to 10 and the molar ratio between isopropanol and propylene ranges from 10 to 0.01.

2. The process according to claim 1, wherein the catalyst is also **characterised by** a total extra-zeolitic porosity volume greater than or equal to 0.80 ml/g.

3. The process for the alkylation of benzene according to the previous claims, wherein the reaction is carried out at a temperature preferably ranging from 150 to 230°C.

4. The process for the alkylation of benzene according to claim 1 or 2, wherein the reaction is carried out at a pressure preferably ranging from 1 to 20 bars.

5. The process for the alkylation of benzene according to the claim 1, wherein the reaction is carried out at a molar ratio between benzene and alkylating agent preferably ranging from 4 to 8.

6. The process for the alkylation of benzene according to the claim 1, wherein the reaction is carried out at a molar ratio between isopropanol and propylene preferably ranging from 5 to 0.1.

7. A process for the preparation of phenol via cumene comprising the following steps:
- alkylation of benzene with isopropanol, and optionally propylene, to give cumene and water,
- oxidation of the cumene thus obtained,
- treatment of cumyl hydroperoxide with acids in order to obtain a mixture of phenol and acetone,
- hydrogenation of acetone to isopropanol
**characterized in that** the initial alkylation of benzene is carried out by means of the process according 8) to one or more of the claims from 1 to 6.

8. The process for the alkylation of benzene according to claim 1 or 2, wherein part of the organic phase of the reaction effluent is re-fed to the reaction section itself, after cooling and separation of the organic phase from the aqueous phase in the reaction effluent.

9. The process for the alkylation of benzene according to claims 1 or 2 or 8, wherein the polyalkylated products present in the effluent of the reaction section are separated in a specific fractionation section and sent to a transalkylation section with benzene.

## Patentansprüche

1. Verfahren für die Alkylierung von Benzol mit Isopropanol oder einer Mischung von Isopropanol und Propylen unter Druck- und Temperaturbedingungen, welche der vollständigen Gasphase des im dem Reaktionsabschnitt vorliegenden Gemisches entsprechen, und in Anwesenheit eines Katalysators, der beta-Zeolith und ein anorganisches Bindemittel umfaßt, **gekennzeichnet durch** eine extra-zeolithische Porosität, bestehend für eine Fraktion von wenigstens 25 %, von Poren mit einem Radius von höher als 100 Angström, bei welchem, wenn das Alkylierungsmittel ein Gemisch von Isopropanol und Propylen ist, das Molverhältnis zwischen Benzol und Alkylierungsmittel von 3 bis 10 reicht und das Molverhältnis zwischen Benzol und Isopropanol von 10 bis 0,01 reicht.

2. Verfahren gemäß Anspruch 1, bei welchem der Katalysator ebenfalls durch ein Volumen der gesamten extra-zeolithischen Porosität größer als oder gleich 0,80 ml/g gekennzeichnet ist.

3. Verfahren für die Alkylierung von Benzol gemäß den vorhergehenden Ansprüchen, bei welchem die Reaktion bei einer Temperatur, die von 150 bis 230 °C reicht, durchgeführt wird.

4. Verfahren für die Alkylierung von Benzol gemäß Anspruch 1 oder 2, bei welchem die Reaktion bei einem Druck, der bevorzugt von 1 bis 20 bar reicht, durchgeführt wird.

5. Verfahren für die Alkylierung von Benzol gemäß Anspruch 1, bei welchem die Reaktion bei einem Molverhältnis zwischen Benzol und Alkylierungsmittel, das bevorzugt von 4 bis 8 reicht, durchgeführt wird.

6. Verfahren für die Alkylierung von Benzol gemäß Anspruch 1, bei welchem die Reaktion bei einem Molverhältnis zwischen Isopropanol und Propylen, das bevorzugt von 5 bis 0,1 reicht, durchgeführt wird.

7. Verfahren für die Herstellung von Phenol über Cumol, umfassend die folgenden Stufen:
- Alkylierung von Benzol mit Isopropanol und wahlweise Propylen zum Erhalt von Cumol und Wasser;
- Oxidation des so erhaltenen Cumols,
- Behandlung von Cumolhydroperoxid mit Säuren, um ein Gemisch von Phenol und Aceton zu erhalten,
- Hydrieren von Aceton zu Isopropanol,
**dadurch gekennzeichnet, daß** die anfängliche Alykylierung mittels des Verfahrens gemäß einem oder mehreren der Ansprüche 1 bis 6 durchgeführt wird.

8. Verfahren für die Alkylierung von Benzol gemäß Anspruch 1 oder 2, bei welchem Teil der organischen Phase der Reaktionsaustrittsströmung zu dem Reaktionsabschnitt selbst nach Kühlen und Abtrennen der organischen Phase von der wässrigen Phase in der Reaktionsaustrittsströmung rückgeführt wird.

9. Verfahren für die Alkylierung von Benzol gemäß Anspruch 1 oder 2 oder 8, bei welchem die in der Austrittsströmung des Reaktionsabschnittes vorliegenden polylakylierten Produkte in einem getrennten Fraktionierungsabschnitt abgetrennt und zu einem Transalkylierungsabschnitt mit Benzol geschickt werden.

## Revendications

1. Procédé pour l'alkylation du benzène avec de l'isopropanol ou un mélange d'isopropanol et de propylène, dans des conditions de pression et de température correspondant à une phase gazeuse complète du mélange dans la section de réaction et en présence d'un catalyseur comprenant une zéolite bêta et d'un liant inorganique **caractérisé par** une porosité extra-zéolitique consistant, pour une fraction d'au moins 25%, en des pores ayant un rayon supérieur à 100 Angstrom, dans lequel, lorsque l'agent alkylant est un mélange d'isopropanol et de propylène, le rapport molaire entre le benzène et l'agent alkylant est de 3 à 10 et le rapport molaire entre l'isopropanol et le propylène est de 10 à 0,01.

2. Procédé selon la revendication 1, dans lequel le catalyseur est également **caractérisé par** un volume de porosité extra-zéolitique total supérieur ou égal à 0,80 mL/g.

3. Procédé pour l'alkylation du benzène selon les revendications précédentes, dans lequel la réaction est réalisée à une température allant de préférence de 150°C à 230°C.

4. Procédé pour l'alkylation du benzène selon la revendication 1 ou 2, dans lequel la réaction est réalisée sous une pression allant de préférence de 1 bar à 20 bars.

5. Procédé pour l'alkylation du benzène selon la revendication 1, dans lequel la réaction est réalisée à un rapport molaire entre le benzène et l'agent alkylant allant de préférence de 4 à 8.

6. Procédé pour l'alkylation du benzène selon la revendication 1, dans lequel la réaction est réalisée à un rapport molaire entre l'isopropanol et le propylène allant de préférence de 5 à 0,1.

7. Procédé de préparation de phénol par l'intermédiaire du cumène comprenant les étapes suivantes :
- alkylation du benzène avec de l'isopropanol, et éventuellement du propylène, pour obtenir du cumène et de l'eau,
- oxydation du cumène ainsi obtenu,
- traitement de l'hydroperoxyde de cumyle avec des acides afin d'obtenir un mélange de phénol et d'acétone,
- hydrogénation de l'acétone en isopropanol
**caractérisé en ce que** l'alkylation initiale est réalisée au moyen du procédé selon une ou plusieurs des revendications 1 à 6.

8. Procédé pour l'alkylation du benzène selon la revendication 1 ou 2, dans lequel une partie de la phase organique de l'effluent de la réaction est réintroduite dans la section de réaction elle-même, après avoir refroidie et séparée la phase organique de la phase aqueuse dans l'effluent de la réaction.

9. Procédé pour l'alkylation du benzène selon les revendications 1, 2 ou 8, dans lequel les produits polyalkylés présents dans l'effluent de la section de réaction sont séparés dans une section de fractionnement spécifique et sont envoyés dans une section de transalkylation avec le benzène.
